(19) Europäisches Patentamt
European Patent Office
Office européen des brevets

(11) Veröffentlichungsnummer : **0 090 087**
**B1**

(12) **EUROPÄISCHE PATENTSCHRIFT**

(45) Veröffentlichungstag der Patentschrift : **15.05.85**

(51) Int. Cl.⁴ : **C 07 C148/04**, C 07 B 57/00

(21) Anmeldenummer : **82111554.0**

(22) Anmeldetag : **13.12.82**

(54) **Verfahren zur Gewinnung von S-(Carboxymethyl)-(R)-cystein und S-(Carboxymethyl)-(S)-cystein.**

(30) Priorität : **26.03.82 DE 3211127**

(43) Veröffentlichungstag der Anmeldung : **05.10.83 Patentblatt 83/40**

(45) Bekanntmachung des Hinweises auf die Patenterteilung : **15.05.85 Patentblatt 85/20**

(84) Benannte Vertragsstaaten : **BE CH DE FR GB LI NL SE**

(56) Entgegenhaltungen :
**CH-A- 401 082**
**H.-G. BOIT: "Beilsteins Handbuch der organischen Chemie", 4. Auflage, drittes Ergänzungswerk, Band 4, Teil II, 1963, Seiten 1591-1592, Springer Verlag, Heidelberg, DE.**

(73) Patentinhaber : **Degussa Aktiengesellschaft**
**Weissfrauenstrasse 9**
**D-6000 Frankfurt am Main 1 (DE)**

(72) Erfinder : **Kleemann, Axel, Dr., Dipl.-Chem.**
**Greifenhagenstrasse**
**D-6450 Hanau am Main 9 (DE)**
Erfinder : **Martens, Jürgen, Dr., Dipl.-Chem.**
**Hochstrasse 10**
**D-8755 Alzenau (DE)**
Erfinder : **Weigel, Horst**
**Odenwaldstrasse 56**
**D-6458 Rodenbach (DE)**

**EP 0 090 087 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist ein Verfahren zur Gewinnung von S-(Carboxymethyl)-(R)-cystein und S-(Carboxymethyl)-(S)-cystein aus einem Gemisch der beiden Enantiomeren.

S-(Carboxymethyl)-cystein, insbesondere das (R)-Enantiomere, besitzt Bedeutung als pharmazeutischer Wirkstoff und wird auch in der kosmetischen Industrie, beispielsweise bei der Herstellung von Haarfestigern, eingesetzt.

Lange Zeit wurde S-(Carboxymethyl)-(R)-cystein ausgehend von natürlichen keratinhaltigen Rohstoffen gewonnen. Diese Rohstoffe wurden hydrolysiert und auf (R)-Cystein aufgearbeitet, welches dann anschließend nach dem aus J. Org. Chem. *16* (1951), Seiten 749 bis 753, bekannten Verfahren in alkalischem Medium mit Chloressigsäure zum S-(Carboxymethyl)-(R)-cystein umgesetzt werden konnte. Geeignete natürliche Rohstoffe stehen jedoch nur in begrenztem Umfang zur Verfügung.

Inzwischen ist aber synthetisches (R S)-Cystein, beispielsweise nach dem aus Angew. Chem. *93* (1981), Seiten 680 und 683, bekannten Verfahren über 2,2-Dimethyl-3-thiazolin und 2,2-Dimethylthiazolidin-4-carbonitril, leicht zugänglich. Seine Umsetzung mit Chloressigsäure liefert dann S-(Carboxymethyl)-(R,S)-cystein. Zur Auftrennung dieses Racemats in die beiden optisch aktiven Enantiomeren besteht ein Bedarf nach einem leistungsfähigen Trennverfahren.

Das erfindungsgemäße Verfahren ist nun dadurch gekennzeichnet, daß man das Gemisch der beiden Enantiomeren in Gegenwart von soviel Ammoniak in Wasser auflöst, daß die gebildete Lösung der Ammoniumsalze einen pH-Wert zwischen 6 und 9 aufweist, in der Lösung der Ammoniumsalze einen Zustand der Übersättigung herbeiführt, durch Zugabe von Impfkristallen des Ammoniumsalzes eines der beiden Enantiomeren — sofern das Ausgangsgemisch eines der beiden Enantiomeren im Überschuß enthält, des Ammoniumsalzes dieses Enantiomeren — das Ammoniumsalz eines der beiden Enantiomeren zur Kristallisation bringt, die ausgeschiedenen Kristalle abtrennt, durch Zugabe von Impfkristallen des Ammoniumsalzes des jeweils anderen Enantiomeren zu der verbleibenden Mutterlauge das Ammoniumsalz dieses anderen Enantiomeren ebenfalls zur Kristallisation bringt, die ausgeschiedenen Kristalle wiederum abtrennt und schließlich aus den Ammoniumsalzen das jeweilige S-(Carboxymethyl)-cystein freisetzt.

Zur Durchführung des erfindungsgemäßen Verfahrens wird das aufzutrennende Gemisch aus S-(Carboxylmethyl)-(R)-cystein und S-(Carboxymethyl)-(S)-cystein entweder in wässrigem Ammoniak aufgelöst oder in Wasser suspendiert und durch Begasen mit gasförmigem Ammoniak in Lösung gebracht. In jedem Falle ist die Menge des Ammoniaks so zu bemessen, daß die gebildete Lösung der Ammoniumsalze einen pH-Wert zwischen 6 und 9, vorzugsweise zwischen 7 und 8,5 aufweist. Um von vornherein eine möglichst konzentrierte Lösung der Ammoniumsalze zu erhalten, kann es zweckmäßig sein, den Lösungsvorgang bei einer erhöhten Temperatur zwischen 25 und 70 °C vorzunehmen.

Anschließend wird dann in der Lösung der Ammoniumsalze ein Zustand der Übersättigung herbeigeführt. Dies kann je nach den Umständen durch Einengen oder Abkühlen der Lösung, durch Zugabe eines mit Wasser mischbaren organischen Lösungsmittels, insbesondere Methanol oder Ethanol, oder durch eine Kombination von mehreren dieser Maßnahmen geschehen.

Dann wird durch Zugabe von Impfkristallen des Ammoniumsalzes eines der beiden Enantiomeren das Ammoniumsalz dieses Enantiomeren zur Kristallisation gebracht. Wenn das Ausgangsgemisch ein echtes Racemat ist, also die beiden Enantiomeren im Verhältnis 1 : 1 enthält, kann zum Animpfen das Ammoniumsalz des S-(Carboxymethyl)-(R)-cysteins oder des S-(Carboxymethyl)-(S)-cysteins verwendet werden. Enthält dagegen das Ausgangsgemisch bereits eines der beiden Enantiomeren im Überschuß, so muß zum Animpfen das Ammoniumsalz des im Überschuß vorhandenen Enantiomeren verwendet werden.

Die ausgeschiedenen Kristalle werden, zweckmäßig nach einigem Stehen, abgetrennt. Die Abtrennung kann in üblicher Weise, z. B. durch Filtration, Zentrifugieren oder Dekantieren der Mutterlauge erfolgen. Die rohen Kristalle können anschließend weiter gereinigt werden, beispeilsweise durch Umkristallisation aus wässrigem Methanol, gegebenenfalls unter Zugabe von Impfkristallen des betreffenden optisch reinen Ammoniumsalzes.

Zu der nach der Abtrennung des Ammoniumsalzes des ersten Enantiomeren verbleibenden Mutterlauge, die einen Überschuß des Ammoniumsalzes des anderen Enantiomeren enthält, werden nun Impfkristalle des Ammoniumsalzes dieses anderen Enantiomeren zugegeben, wodurch das Ammoniumsalz dieses anderen Enantiomeren ebenfalls zur Kristallisation gebracht wird. Die ausgeschiedenen Kristalle werden wiederum abgetrennt und gegebenenfalls weiter gereinigt wie oben beschrieben.

Besonders vorteilhaft ist es, wenn man der nach der Abtrennung des Ammoniumsalzes des ersten Enantiomeren verbleibenden Mutterlauge vor der Weiterbehandlung zusätzliches racemisches S-(Carboxymethyl)-cystein, entweder in Form des Ammoniumsalzes oder unter gleichzeitiger Zugabe von Ammoniak in solcher Menge, daß die gebildete Lösung wieder einen pH-Wert in dem oben genannten Bereich aufweist, zusetzt.

Schließlich wird aus den beiden abgetrennten und gegebenenfalls weiter gereinigten Ammoniumsalzen getrennt das jeweilige S-(Carboxymethyl)-cystein freigesetzt. Dies kann beispielsweise dadurch geschehen, daß man das Ammoniumsalz in Wasser auflöst und mit einer Mineralsäure, z. B. Salzsäure, einen pH-Wert in der Nähe des isoelektrischen Punktes von S-(Carboxymethyl)-cystein, also

einen pH-Wert von etwa 2 bis 3, einstellt. Das dann ausfallende S-(Carboxymethyl)-cystein wird abgetrennt und getrocknet. Alternativ dazu kann das Ammoniumsalz auch mit Hilfe eines Ionenaustauschers in das freie S-(Carboxymethyl)-cystein umgewandelt werden.

Besonders vorteilhaft gestaltet sich das erfindungsgemäße Verfahren, wenn man es in einer Vielzahl von Stufen entsprechend dem nachfolgenden Schema durchführt. In diesem Schema bedeuten « (RS)-SCC.$NH_3$ » das Ammoniumsalz des racemischen S-(Carboxymethyl)-cysteins, « (R)-SCC.$NH_3$ » das Ammoniumsalz des S-(Carboxymethyl)-(R)-cysteins und « (S)-SCC.$NH_3$ » das Ammoniumsalz des S-(Carboxymethyl)-(S)-cysteins.

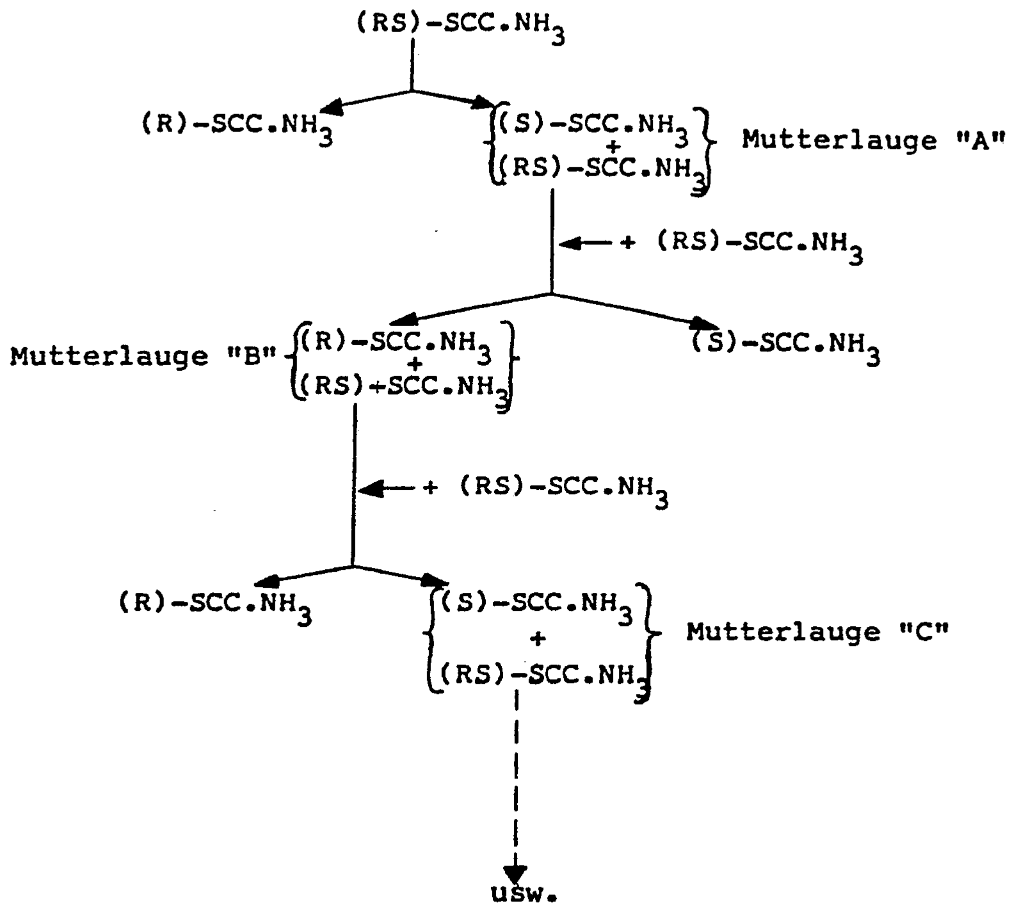


Die gegebenenfalls erforderliche Umkristallisation des jeweils abgetrennten Ammoniumsalzes ist der besseren Übersichtlichkeit halber in diesem Schema nicht berücksichtigt. Wird eine Umkristallisation vorgenommen, so wird zweckmäßigerweise die dabei anfallende Mutterlauge zu einer passenden Mutterlauge des Trennungsganges zugegeben, also z. B. die Mutterlauge aus der Umkristallisation von (R)-SCC.$NH_3$ zu Mutterlauge « B » usw. und die Mutterlauge aus der Umkristallisation von (S)-SCC.$NH_3$ zu Mutterlauge « A », « C » usw. Sofern der Zustand der Übersättigung in der Lösung der Ammoniumsalze durch Zugabe eines organischen Lösungsmittels herbeigeführt wurde und/oder bei der Umkristallisation des jeweils abgetrennten Ammoniumsalzes ein solches organisches Lösungsmittel mitverwendet wurde, empfiehlt es sich, vor der Weiterverarbeitung der gegebenenfalls vereinigten Mutterlaugen die enthaltenen organischen Lösungsmittel durch Destillation abzutrennen.

Bei der geschilderten vielstufigen Verfahrensweise werden letztlich die Ammoniumsalze der beiden Enantiomeren vollständig voneinander getrennt, was zu einer entsprechend hohen Ausbeute an den beiden optisch aktiven S-(Carboxymethyl)-cysteinen führt.

Das erfindungsgemäße Verfahren kann auch bei der Reinigung von rohem S-(Carboxymethyl)-(R)-cystein oder S-(Carboxymethyl)-(S)-cystein mit noch unbefriedigender optischer Reinheit angewandt werden, welche das eine der beiden Enantiomeren bereits im Überschuß enthalten. Diese zusätzliche Reinigung besitzt besondere Bedeutung für die Gewinnung von reinem S-(Carboxymethyl)-(R)-cystein, das aus (R,R)-cystin bzw. (R)-cystein hergestellt wurde. Denn bei der Hydrolyse von natürlichen keratinhaltigen Rohstoffen tritt in beträchtlichem Maße Racemisierung des natürlichen Cystins bzw. Cysteins ein.

Andererseits kann natürlich auch das nach dem erfindungsgemäßen Verfahren gewonnene und derzeit noch weniger gefragte S-(Carboxymethyl)-(S)-cystein einer racemisierenden Behandlung unter-

worfen werden und dann als Ausgangsmaterial für die Gewinnung von weiterem S-(Carboxymethyl)-(R)-cystein dienen. Die Racemisierung kann beispielsweise durch Kochen in wässriger salzsaurer Lösung in Gegenwart von Salicylaldehyd bewirkt werden.

Die Erfindung wird durch die nachfolgenden Beispiele näher erläutert. Prozentangaben bedeuten, sofern nicht anders angegeben, Gewichtsprozente.

Das in den Beispielen eingesetzte S-(Carboxymethyl)-(R S)-cystein wurde wie folgt hergestellt : 140 g (1 Mol) synthetisches (R S)-Cystein-hydrochlorid und 160 g (4 Mol) Natriumhydroxid wurden in 1 000 ml wasser gelöst. Der Lösung wurden zunächst 3 g Natriumhydrogensulfit und dann im Verlaufe von 45 Minuten 95 g (1 Mol) Monochloressigsäure zugesetzt. Die Temperatur des Reaktionsgemisches wurde währenddessen auf 20 °C und anschließend noch 3 Stunden lang auf 25 bis 30 °C gehalten. Schließlich wurde durch Zugabe von konzentrierter wässriger Chlorwasserstoffsäure der pH auf 3,0 eingestellt, worauf sich das S-(Carboxymethyl)-(R S)-cystein ausschied. Es wurde bei 10 °C abfiltriert, solange mit Wasser gewaschen, bis das Filtrat frei von Chloridionen war, und unter vermindertem Druck bei 150 °C getrocknet. Die Ausbeute betrug 173 g, entsprechend 97 % der Theorie, der Schmelzpunkt betrug 188 bis 192 °C (Zersetzung).

Elementaranalyse :

| | | | | |
|---|---|---|---|---|
| Gefunden : | C 33,70 % | H 5,07 % | N 7,91 % | S 17,79 % |
| Berechnet : | C 33,51 % | H 5,06 % | N 7,82 % | S 17,89 % |

Die in den Beispielen gewonnenen optisch aktiven Enantiomeren wurden jeweils auf ihren spezifischen Drehwert $[\alpha]_D^{20}$ in Grad · cm³/dm · g untersucht.

## Beispiel 1

40 g S-(Carboxymethyl)-(R S)-cystein werden mit 40 ml Wasser vermischt und unter Erwärmen auf 60 °C mit 24 %-iger wässriger Ammoniaklösung versetzt, bis bei pH 8,5 eine klare Lösung entsteht. Nach Zugabe von 70 ml Methylalkohol wird auf 20 °C abgekühlt und mit 220 mg des Ammoniumsalzes von S-(Carboxymethyl)-(R)-cystein angeimpft. Nach 70 Minuten wird abgesaugt. Nach dem Umkristallisieren des Rückstandes aus 70 %igem wässrigen Methylalkohol erhält man 3,1 g des Ammoniumsalzes von S-(Carboxymethyl)-(R)-cystein mit einem Drehwert $[\alpha]_D^{20}$ = – 29,8° (c = 5, Wasser).

Das Salz wird in 20 ml Wasser gelöst und mit Salzsäure ein pH von 2,5 eingestellt. Nach Absaugen, Waschen mit 100 ml Wasser und Trocknen verbleiben 2,6 g S-(Carboxymethyl)-(R)-cystein mit einem Drehwert $[\alpha]_D^{20}$ = – 34,4° (c = 5, Wasser, Natronlauge bis pH 6).

In dem nach der Abtrennung des Ammoniumsalzes von S-(Carboxymethyl)-(R)-cystein verbleibenden Filtrat werden unter Erwärmen auf 35 °C 8 g des Ammoniumsalzes von S-(Carboxymethyl)-(R S)-cystein gelöst. Die klare Lösung wird auf 20 °C abgekühlt und mit einigen Kristallen des Ammoniumsalzes von S-(Carboxymethyl)-(S)-cystein angeimpft. Nach 35 Minuten werden durch Absaugen 7,8 g des Ammoniumsalzes von S-(Carboxymethyl)-(S)-cystein mit einem Drehwert $[\alpha]_D^{20}$ = + 20,7° (c = 5, Wasser) erhalten.

Das Salz wird aus wässrigem Methylalkohol umkristallisiert, dann in 20 ml Wasser gelöst. Die Lösung wird mit Salzsäure auf pH 2,5 angesäuert. Durch Absaugen, Waschen mit 200 ml Wasser und Trocknen werden 4,8 g S-(Carboxymethyl)-(S)-cystein mit einem Drehwert $[\alpha]_D^{20}$ = + 34,0° (c = 5, Wasser, Natronlauge bis pH 6) erhalten.

## Beispiel 2

Eine Mischung aus 75 g S-(Carboxymethyl)-(R S)-cystein und 5 g S-(Carboxymethyl)-(R)-cystein wird unter Erwärmen auf 60 °C in 70 ml Wasser und 42 ml 24 %iger wässriger Ammoniaklösung gelöst. Die klare Lösung mit einem pH von 8,0 wird mit 140 ml Methylalkohol versetzt und auf 25 °C abgekühlt. Unter langsamen Rühren der übersättigten klaren Lösung wird 200 mg des Ammoniumsalzes von S-(Carboxymethyl)-(R)-cystein angeimpft. Nach 25 Minuten werden die groben farblosen Kristalle abgesaugt und bei 50 °C unter vermindertem Druck getrocknet. Man erhält 19,1 g des Ammoniumsalzes von S-(Carboxymethyl)-(R)-cystein mit einem Drehwert $[\alpha]_D^{20}$ = – 25,6° (c = 5, Wasser).

Durch Umkristallisieren aus 80 %igem wässrigem Methylalkohol steigt der Drehwert auf $[\alpha]_D^{20}$ = – 30,6° an.

Das Salz wird in 50 ml Wasser gelöst und die Lösung mit Salzsäure auf pH 2,5 angesäuert. Die ausgeschiedenen Kristalle werden abgesaugt, mit Wasser gewaschen und getrocknet. Man erhält 13,9 g S-(Carboxymethyl)-(R)-cystein mit einem Drehwert $[\alpha]_D^{20}$ = – 35,1° (c = 5, Wasser, Natronlauge bis pH 6).

Das nach der Abtrennung des Ammoniumsalzes von S-(Carboxymethyl)-(R)-cystein verbleibende Filtrat wird mit 100 g des Ammoniumsalzes von S-(Carboxymethyl)-(S)-cystein angeimpft und bleibt 20 Minuten stehen. Durch Absaugen und Trocknen werden 8,6 g des Ammoniumsalzes von S-(Carboxymethyl)-(S)-cystein mit einem Drehwert $[\alpha]_D^{20}$ = + 24,8° (c = 5, Wasser) erhalten.

Durch Umkristallisieren aus 80 %igem wässrigem Methylalkohol steigt der Drehwert auf $[\alpha]_D^{20}$ = + 30,6° an.

Das Salz wird in 40 ml Wasser gelöst und die Lösung mit Salzsäure auf pH 2,5 angesäuert. Die ausgeschiedenen Kristalle werden abgesaugt, mit Wasser gewaschen und getrocknet. Man erhält 6,1 g S-(Carboxymethyl)-(S)-cystein mit einem Drehwert $[\alpha]_D^{20} = + 35,0°$ (c = 5, Wasser, Natronlauge bis pH 6).

## Beispiel 3

Eine Mischung aus 74 g S-(Carboxymethyl)-(R S)-cystein und 6 g S-(Carboxymethyl)-(S)-cystein wird unter Erwärmen auf 50 °C mit 70 ml Wasser verrührt und mit 24 %iger wässriger Ammoniaklösung versetzt, bis bei pH 7,8 eine klare Lösung entsteht. Nach Zugabe von 140 ml Methylalkohol wird die Lösung auf 23 °C abgekühlt, mit 60 mg des Ammoniumsalzes von S-(Carboxymethyl)-(S)-cystein angeimpft und 20 Minuten ohne Rühren stehen gelassen. Durch anschließendes Absaugen und Trocknen werden 20,4 g des Ammoniumsalzes von S-(Carboxymethyl)-(S)-cystein mit einem Drehwert $[\alpha]_D^{20} = + 23,8°$ (c = 5, Wasser) erhalten.

Durch Umkristallisieren aus 80 %igem wässrigem Methylalkohol steigt der Drehwert auf $[\alpha]_D^{20} = + 30,8°$ an.

Elementaranalyse : $C_5H_{12}N_2O_4S$

| | | | | |
|---|---|---|---|---|
| Gefunden : | C 30,48 % | H 6,21 % | N 14,10 % | S 16,30 % |
| Berechnet : | C 30,60 % | H 6,16 % | N 14,28 % | S 16,34 % |

Das Salz wird in 50 ml Wasser gelöst und die Lösung mit Salzsäure auf pH 2,5 angesäuert. Nach dem Absaugen, Waschen mit 90 ml Wasser und Trocknen werden 12,9 g S-(Carboxymethyl)-(S)-cystein mit einem Drehwert $[\alpha]_D^{20} = + 34,8°$ (c = 5, Wasser, Natronlauge bis pH 6) erhalten.

Das nach der Abtrennung des Ammoniumsalzes von S-(Carboxymethyl)-(S)-cystein verbleibende Filtrat wird mit 20 g S-(Carboxymethyl)-(R S)-cystein versetzt, auf 45 °C erwärmt und durch Einleiten von gasförmigem Ammoniak bis pH 7,8 in eine klare Lösung überführt. Nach Abkühlen auf 28 °C und Animpfen der übersättigten Lösung mit 50 mg des Ammoniumsalzes von S-(Carboxymethyl)-(R)-cystein kristallisieren innerhalb von 25 Minuten 19,6 g des Ammoniumsalzes von S-(Carboxymethyl)-(R)-cystein mit einem Drehwert $[\alpha]_D^{20} = - 21,6°$ (c = 5, Wasser) aus.

Der Drehwert steigt nach Umkristallisieren aus 80 %igem wässrigem Methylalkohol auf $[\alpha]_D^{20} = - 30,1°$ an.

Das Salz wird in 50 ml Wasser gelöst und die Lösung mit Salzsäure auf pH 2,5 angesäuert. Durch Absaugen, Waschen mit 40 ml Wasser und trocknen erhält man 13,3 g S-(Carboxymethyl)-(R)-cystein mit einem Drehwert $[\alpha]_D^{20} = - 35,1°$ (c = 5, Wasser, Natronlauge bis pH 6).

Elementaranalyse :

| | | | | |
|---|---|---|---|---|
| Gefunden : | C 33,65 % | H 5,12 % | N 7,94 % | S 17,82 % |
| Berechnet : | C 33,51 % | H 5,06 % | N 7,82 % | S 17,89 % |

## Beispiel 4

Eine Mischung aus 91 g S-(Carboxymethyl)-(R S)-cystein und 9 g S-(Carboxymethyl)-(S)-cystein wird in einem Wasserbad von 50 °C mit 40 ml Wasser versetzt und mit konzentrierter wässriger Ammoniaklösung verrührt, bis bei pH 7,6 eine klare Lösung entsteht. Diese Lösung wird auf 25 °C abgekühlt und mit 100 mg des Ammoniumsalzes von S-(Carboxymethyl)-(S)-cystein angeimpft. Nach 45 Minuten wird der Kristallbrei abgesaugt und man erhält 23,3 g des Ammoniumsalzes von S-(Carboxymethyl)-(S)-cystein mit einem Drehwert $[\alpha]_D^{20} = + 24,2°$ (c = 5, Wasser) und die Mutterlauge « A ».

Das Salz wird aus 80 %igem wässrigem Methylalkohol umkristallisiert, in 50 ml Wasser gelöst und die Lösung mit Salzsäure auf pH 2,5 angesäuert. Nach Absaugen, Waschen mit 75 ml Wasser und Trocknen erhält man 16,0 g S-(Carboxymethyl)-(S)-cystein mit einem Drehwert $[\alpha]_D^{20} = + 34,8°$ (c = 5, Wasser, Natronlauge bis pH 6).

Die Mutterlauge « A » wird auf 50 °C erwärmt, mit 20 g S-(Carboxymethyl)-(R S)-cystein versetzt und mit konzentrierter wässriger Ammoniaklösung auf pH 7,6 eingestellt. Nach dem Abkühlen auf 25 °C wird jetzt mit 100 mg des Ammoniumsalzes von S-(Carboxymethyl)-(R)-cystein angeimpft.

Nach 45 Minuten wird erneut abgesaugt und es werden 20,5 g des Ammoniumsalzes von S-(Carboxymethyl)-(R)-cystein mit einem Drehwert $[\alpha]_D^{20} = - 23,9°$ (c = 5, Wasser) und die Mutterlauge « B » erhalten.

Das Salz wird aus 80 %igem wässrigem Methylalkohol umkristallisiert, in 50 ml Wasser gelöst und die Lösung mit Salzsäure auf pH 2,5 angesäuert. Nach Absaugen, Waschen mit 50 ml Wasser und Trocknen erhält man 14,2 g S-(Carboxymethyl)-(R)-cystein mit einem Drehwert $[\alpha]_D^{20} = - 35,0°$ (c = 5, Wasser, Natronlauge bis pH 6).

Die Mutterlauge « B » wird wieder auf 50 °C erwärmt, mit 20 g S-(Carboxymethyl)-(R S)-cystein versetzt und mit konzentrierter wässriger Ammoniaklösung auf pH 7,6 eingestellt. Nach dem Abkühlen auf 25 °C wird diesmal wieder mit 100 mg des Ammoniumsalzes von S-(Carboxymethyl)-(S)-cystein angeimpft. Nach 45 Minuten wird erneut abgesaugt und es werden weitere 22,6 g des Ammoniumsalzes

von S-(Carboxymethyl)-(S)-cystein mit einem Drehwert $[\alpha]_D^{20}$ = + 23,0° (c = 5, Wasser) und die Mutterlauge « C » erhalten.

Das Salz wird wieder aus 80 %igem, wässrigem Methylalkohol umkristallisiert, in 50 ml Wasser gelöst und die Lösung mit Salzsäure auf pH 2,5 angesäuert. Nach Absaugen, Waschen mit 150 ml Wasser und Trocknen erhält man weitere 15,5 g S-(Carboxymethyl)-(S)-cystein mit einem Drehwert $[\alpha]_D^{20}$ = + 35,1° (c = 5, Wasser, Natronlauge bis pH 6).

Mit der Mutterlauge « C » wird ebenso verfahren wie oben mit der Mutterlauge « A » und es werden weitere 14,5 g S-(Carboxymethyl)-(R)-cystein erhalten.

**Patentansprüche**

1. Verfahren zur Gewinnung von S-(Carboxymethyl)-(R)-cystein und S-(Carboxymethyl)-(S)-cystein aus einem Gemisch der beiden Enantiomeren, dadurch gekennzeichnet, daß man das Gemisch in Gegenwart von soviel Ammoniak in Wasser auflöst, daß die gebildete Lösung der Ammoniumsalze eine pH-Wert zwischen 6 und 9 aufweist, in der Lösung der Ammoniumsalze einen Zustand der Übersättigung herbeiführt, durch Zugabe von Impfkristallen des Ammoniumsalzes eines der beiden Enantiomeren — sofern das Ausgangsgemisch eines der beiden Enantiomeren im Überschuß enthält, des Ammoniumsalzes dieses Enantiomeren — das Ammoniumsalz eines der beiden Enantiomeren zur Kristallisation bringt, die ausgeschiedenen Kristalle abtrennt, durch Zugabe von Impfkristallen des Ammoniumsalzes des jeweils anderen Enantiomeren zu der verbleibenden Mutterlauge das Ammoniumsalz dieses Enantiomeren ebenfalls zur Kristallisation bringt, die ausgeschiedenen Kristalle wiederum abtrennt und schließlich aus den Ammoniumsalzen das jeweilige S-(Carboxymethyl)-cystein freisetzt.

2. Verfahren nach Anspruch 1, dadurch gekennzeichnet, daß man den Ammoniak in Form einer wässrigen Lösung einsetzt.

3. Verfahren nach Anspruch 1, dadurch gekennzeichnet, daß man den Ammoniak als gasförmigen Ammoniak einsetzt.

4. Verfahren nach einem der Ansprüche 1 bis 3, dadurch gekennzeichnet, daß man den Zustand der Übersättigung in der Lösung der Ammoniumsalze durch Einengen herbeiführt.

5. Verfahren nach einem der Ansprüche 1 bis 3, dadurch gekennzeichnet, daß man den Zustand der Übersättigung in der Lösung der Ammoniumsalze durch Abkühlen herbeiführt.

6. Verfahren nach einem der Ansprüche 1 bis 3, dadurch gekennzeichnet, daß man den Zustand der Übersattigung in der Lösung der Ammoniumsalze durch Zugabe eines mit Wasser mischbaren organischen Lösungsmittels herbeiführt.

7. Verfahren nach einem der Ansprüche 1 bis 6, dadurch gekennzeichnet, daß man der nach dem Abtrennen des Ammoniumsalzes des ersten Enantiomeren verbleibenden Mutterlauge vor der Weiterbehandlung racemisches S-(Carboxymethyl)-cystein und Ammoniak oder das Ammoniumsalz des racemischen S-(Carboxymethyl)-cysteins zusetzt.

**Claims**

1. Process for the recovery of S-(carboxymethyl)-(R)-cysteine and S-(carboxymethyl)-(S)-cysteine from a mixture of the two enantiomers, characterised in that the mixture is dissolved in water in the presence of a sufficient quantity of ammonia for the resulting solution of the ammonium salts to have a pH-value of from 6 to 9, a state of supersaturation in the solution of the ammonium salts is produced, the ammonium salt of one of the two enantiomers is crystallized by addition of seed crystals of the ammonium salt of this enantiomer — provided that when the starting mixture contains one of the two enantiomers in excess, the seed crystals are of the ammonium salt of this enantiomer — the precipitated crystals are separated off, the ammonium salt of the other enantiomer is also crystallized by addition of seed crystals of the ammonium salt of this enantiomer to the remaining mother liquor, the precipitated crystals are again separated off and finally the respective S-(carboxymethyl)-cysteines are freed from the ammonium salts.

2. Process according to claim 1, characterised in that the ammonia is used in the form of an aqueous solution.

3. Process according to claim 1, characterised in that the ammonia is used as gaseous ammonia.

4. Process according to one of claims 1 to 3, characterised in that the state of supersaturation in the solution of the ammonium salts is caused by concentration.

5. Process according to one of claims 1 to 3, characterised in that the state of supersaturation in the solution of the ammonium salts is caused by cooling.

6. Process according to one of claims 1 to 3, characterised in that the state of supersaturation in the solution of the ammonium salts is caused by addition of a water-miscible organic solvent.

7. Process according to one of claims 1 to 6, characterised in that, before further treatment, racemic S-(carboxymethyl)-cysteine and ammonia or the ammonium salt of the racemic S-(carboxymethyl)-cysteine is added to the mother liquor remaining after separation of the ammonium salt of the first enantiomer.

**Revendications**

1. Procédé d'obtention de S-(carboxyméthyl)-(R)-cystéine et de S-(carboxyméthyl)-(S)-cystéine, à partir d'un mélange des deux énantiomères, procédé caractérisé en ce que le mélange est dissous dans l'eau en présence d'une quantité d'ammoniac telle que la solution de sel d'ammonium formée présente un pH compris entre 6 et 9, la solution de sel d'ammonium est amenée à l'état sursaturé, que par introduction de germes cristallins du sel d'ammonium d'un des deux énantiomères — dans la mesure où le mélange de départ contient un des deux énantiomères en excès par rapport à l'autre, ce sera le sel d'ammonium de cet énantiomère — le sel d'ammonium d'un des deux énantiomères est amené à cristalliser, les cristaux déposés sont séparés, que par introduction de germes cristallins du sel d'ammonium de l'autre énantiomère dans la solution mère restante, on amène le sel d'ammonium de cet énantiomère à cristalliser également, les cristaux déposés sont séparés à leur tour et enfin, chaque S-(carboxyméthyl)-cystéine et libérée à partir du sel d'ammonium correspondant.

2. Procédé selon la revendication 1, caractérisé en ce que l'ammoniac est mis en œuvre sous forme de solution aqueuse.

3. Procédé selon la revendication 1, caractérisé en ce que l'ammoniac est mis en œuvre sous forme gazeuse.

4. Procédé selon l'une quelconque des revendications 1 à 3, caractérisé en ce que l'état de sursaturation de la solution de sels d'ammonium.

5. Procédé selon l'une quelconque des revendications 1 à 3, caractérisé en ce que l'état de sursaturation de la solution de sel d'ammonium est obtenu par refroidissement.

6. Procédé selon l'une quelconque des revendications 1 à 3, caractérisé en ce que l'état de sursaturation de la solution de sel d'ammonium est obtenu par addition d'un solvant organique miscible à l'eau.

7. Procédé selon l'une des revendications 1 à 6, caractérisé en ce que dans la solution mère restante après la séparation du sel d'ammonium du premier énantiomère, on introduit, avant de continuer le traitement, de la S-(carboxyméthyl)-cystéine racémique et de l'ammoniac, ou du sel d'ammonium de la S-(carboxyméthyl)-cystéine racémique.